# EUROPÄISCHE PATENTANMELDUNG

(11) **EP 0 995 464 A2**
(43) Veröffentlichungstag der Anmeldung: **26.04.2000**
(21) Anmeldenummer: 98122493.4
(22) Anmeldetag: 30.11.1998
(51) Int. Cl.: A61N 2/06

(54) **Trägeranordnung für elektropolarisierte Elemente, insbesondere für therapeutische Zwecke zur äusseren Anwendung**

(30) Priorität: 18.10.1998 DE 29818506 U
(71) Anmelder: YAKA-BOX, S.L., 07004 Palma de Mallorca (ES)
(72) Erfinder: Jimenez Marin, Pascual, E-07004 Palma de Mallorca (ES)
(74) Vertreter: Dosterschill, Peter, Dr.

(57) **Zusammenfassung**

Die Erfindung betrifft eine Trägeranordnung (1) für elektropolarisierte Elemente, insbesondere für therapeutische Zwecke zur äußeren Anwendung.

Die Trägeranordnung weist zwei elektropolarisierte Elemente (6, 6') und mindestens ein Verbindungselement (2, 4, 3, 5, 4', 3', 2') auf, das die elektropolarisierten Elemente (6, 6') miteinander verbindet. Weiterhin kann die Trägeranordnung ein Befestigungselement (4, 3, 5, 4', 3') aufweisen, mit dem sie an einem Kleidungsstück und/oder an einem Element befestigbar ist, das an oder in der Nähe eines zu therapierenden Körperteils anbringbar ist. Vorzugsweise ist das Verbindungselement als das Befestigungselement ausgebildet.

## Beschreibung

### GEGENSTAND DER ERFINDUNG

Die Erfindung betrifft eine Trägeranordnung für elektropolarisierte Elemente, insbesondere für therapeutische Zwecke zur äußeren Anwendung, nach dem Oberbegriff des Anspruchs 1.

### STAND DER TECHNIK

Es ist eine Mehrzahl von Ringen, Ohrringen und Armbändern bekannt, die als magnetische Körper ausgestaltet sind und für therapeutische Zwecke benutzt werden. Diese dienen der Linderung bzw. der Eliminierung von Schmerzen oder Anomalien, die ihre Ursache in verschiedenen Krankheiten haben, insbesondere Rheumatismus, Artrose und Kreislauferkrankungen.

Ganz allgemein gilt, daß die Armbänder, Ringe und Ohrringe eine elliptische Form mit einer offenen Zone aufweisen, wobei an den freien Enden Abschlußstücke angeordnet sind. In diese Abschlußstücke können Magnete eingefügt werden, die in verschiedenen Legierungen ausgeführt sind und die eine nur vergleichsweise geringe magnetische Flußdichte aufweisen.

Die Armbänder und ähnlichen Gegenstände magnetischer Konfiguration sind aus unterschiedlichen Materialien gefertigt und können eine Mehrzahl von Sektionen bzw. Abschnitte und gleichzeitig unterschiedliche Formen aufweisen. Sie können auch aus mehreren geflochtenen bzw. verlitzten Drähten bestehen.

Es sind magnetische Armbänder bekannt, die Gegenstand des spanischen Gebrauchsmusters Nr. 253.127 sind, und deren tatsächliche Realisierung Gegenstand des spanischen Geschmacksmusters Nr. 99.171 sind.

Das magnetische Armband gemäß spanischem Gebrauchsmuster Nr. 253.127 ist für eine Mehrzahl therapeutischer Zwecke anwendbar. Es hat eine elliptische Form und einen offenen Abschnitt, an dessen freien Enden und nach innen gerichtet Magnete eingefügt sind, die im Kontakt mit der Haut der jeweiligen Person sind, die das Armband trägt.

Das zuvor genannte spanische Geschmacksmuster Nr. 99.171 offenbart ein magnetisches Armband, das aus einem einstückigen Körper gebildet ist. Dieser Körper weist die offene elliptische Konfiguration auf und hat einen rechteckigen Abschnitt, wobei der einstückige offene Körper an seinen Enden und nach innen gerichtet einige halbkreisförmige Ausrüstungen bzw. Abschlüsse aufweist, die aus dem Hauptkörper hervorstehen und die durch zylindrische Magneten geringer Höhe abgeschlossen sind.

Weiterhin ist eine Mehrzahl spanischer Geschmacksmuster und Gebrauchsmuster bekannt, in denen verschiedene Typen von Armbändern, Ohrringen und Ringen beschrieben sind, die an Handgelenken, Fußknöcheln, Ohren oder an Nasenhöhlen ihre besondere Wirkung erzielen.

Die bekannten Gegenstände wie z.B. Armbänder lassen sich jedoch nicht an beliebigen Körperteilen einer Person einsetzen.

Ausgehend von diesem Stand der Technik liegt der Erfindung die Aufgabe zugrunde, eins Anordnung der eingangs genannten Art zu schaffen, die erweiterte Nutzungsmöglichkeiten bietet.

Diese Aufgabe wird erfindungsgemäß mit einer Anordnung gelöst, die in Anspruch 1 definiert ist.

Die erfindungsgemäße Trägeranordnung läßt sich in einfacher Weise an unterschiedlichen Stellen eines zu therapierenden Körpers bzw. Körperteils anbringen. Ein Befestigungselement der erfindungsgemäßen Trägeranordnung erlaubt die einfache Befestigung beispielsweise an Unterwäsche, so daß sich elektropolarisierte Elemente der Trägeranordnung zum Beispiel im Bereich des Unterleibs, insbesondere im Bereich der Eierstöcke, anordnen lassen. Die erfindungsgemäße Trägeranordnung kann zum Beispiel auch in einen Gürtel, in eine Tasche oder in ein Band integriert werden und damit auch im Bereich des Kopfes eingesetzt werden.

Das Befestigungselement ist insbesondere als Klemme bzw. Klipp ausgestaltet und ermöglicht es, die Trägeranordnung in einfacher Weise, mit einem Handgriff, z.B. an eines Kleidungsstück zu befestigen oder nach Gebrauch in ebenso einfacher Weise von diesem Kleidungsstück zu lösen. Zugleich sichert die Ausgestaltung des Befestigungselements eine sichere Befestigung auch während eines Gebrauchs. Die Trägeranordnung kann während des Tags und während der Nacht genutzt werden, ohne daß die Person, die die Trägeranordnung nutzt, irgendwelchen Einschränkungen hinsichtlich ihrer Beweglichkeit unterliegt.

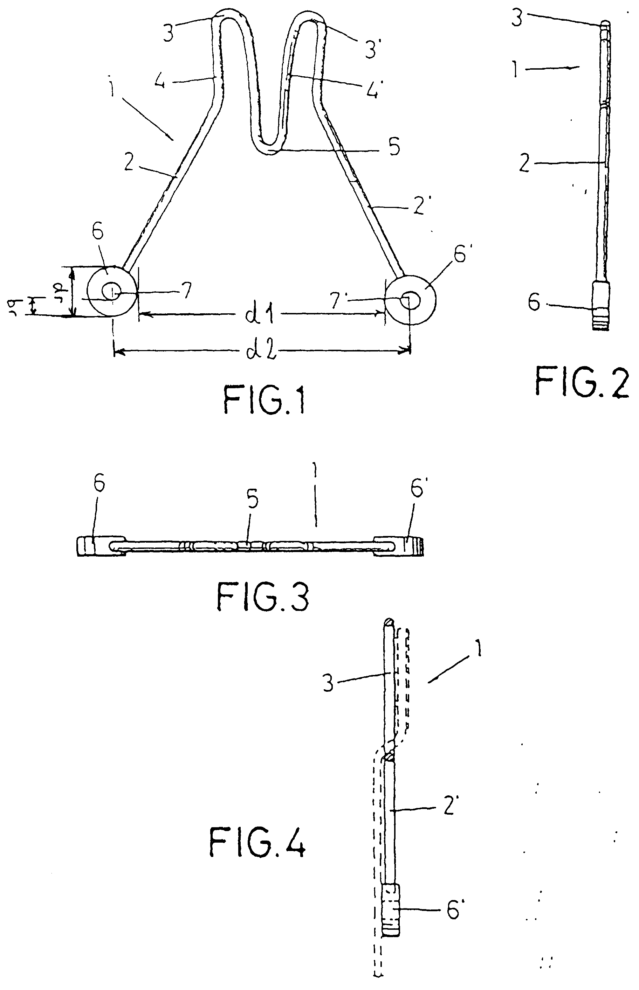

Die erfindungsgemäße Trägeranordnung einschließlich der elektropolarisierten Elemente zeichnet sich auch durch die vergleichsweise flache Ausbildung (siehe Figuren 1 bis 4), durch ein geringes Gewicht und insgesamt durch einen hohen Tragekomfort aus.

Die erfindungsgemäße Trägeranordnung ist robust und unterliegt praktisch keinem Verschleiß. Sie läßt sich in einfacher Weise herstellen und nach Gebrauch in einfacher Wiese z.B. mit Alkohol oder destilliertem Wasser reinigen.

Die erfindungsgemäße Trägeranordnung läßt sich auch z.B. mit einem selbstklebenden Band an dem zu therapierenden Körperteil befestigen, wobei die elektropolarisierten Elemente Kontakt mit dem entsprechenden Körperteil haben. Die elektropolarisierten Körper lassen sich auch in eine Tasche integrieren, wobei die Tasche an der dem zu therapierenden Körperteil zugewandten Seite mindestens eine Öffnung aufweist, die den Kontakt der elektropolarisierten Elemente mit dem zu therapierenden Körperteil ermöglicht.

### BESCHREIBUNG DER ZEICHNUNG

Die Erfindung wird nun anhand der Zeichnung beschrieben.
Es zeigt
- Figur 1: eine Vorderansicht einer ersten Ausführungsbeispiels einer erfindungsgemäßen Trägeranordnung;
- Figur 2: eine Seitenansicht der erfindungsgemäßen Trägeranordnung nach Figur 1;
- Figur 3: eine Ansicht einer horizontal angeordneten erfindungsgemäßen Trägeranordnung nach den Figuren 1 und 2;
- Figur 4: eine Seitenansicht einer erfindungsgemäßen Trägeranordnung, ähnlich wie Figur 2, , wobei in Figur 4 ein Textilträger, an welchem die Trägeranordnung befestigt ist, durch eine gestrichelte Linie dargestellt ist;
- Figuren 5 bis 13: Vorderansichten weiterer Ausführungsbeispiele einer erfindungsgemäßen Trägeranordnung;
- Figuren 14 und 15: Ausführungsbeispiele von Taschen zur Aufnahme elektropolarisierter Elemente bzw. zur Aufnahme einer erfindungsgemäßen Trägeranordnung;
- Figur 16: eine Anordnung zur Messung des magnetischen Flußdichte erfindungsgemäßer Trägeranordnungen; und
- Figuren 17 und 18: Meßdiagramme mit Ergebnissen von Messungen an zwei unterschiedlichen Trägeranordnungen.

Die erfindungsgemäße Trägeranordnung, die in unterschiedlichen Ausgestaltungen in den Figuren 1 bis 13 dargestellt ist, weist elektropolarisierte Elemente 6, 6' (Figur 1) auf, die insbesondere für therapeutische Zwecke zur äußeren Anwendung bestimmt sind.

Die Trägeranordnung 1 ist folgendermaßen ausgestaltet:
Sie weist bei den in den Figuren 1 bis 13 dargestellten Ausführungsbeispielen, zwei elektropolarisierte Elemente 6, 6' auf. Weiterhin weist die Trägeranordnung 1 mindestens ein Verbindungselement 2, 4, 3, 5 (4', 3', 2') auf, das die elektropolarisierten Elemente 6, 6' miteinander verbindet.

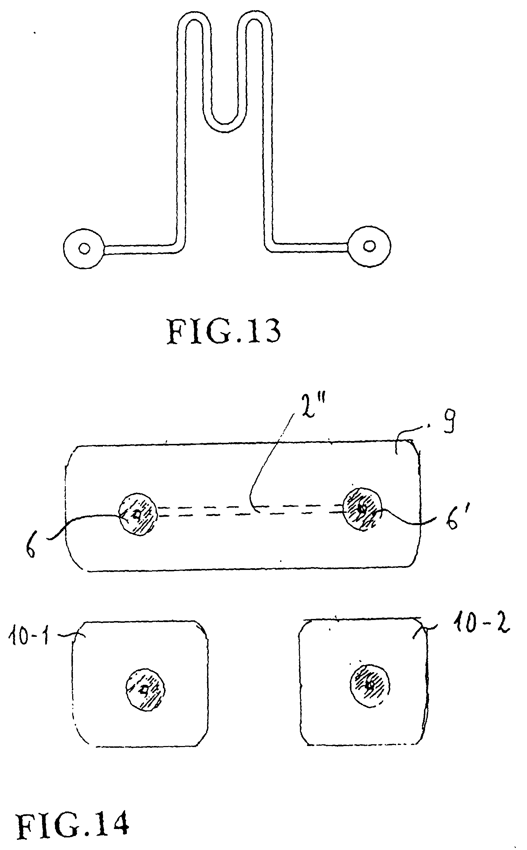

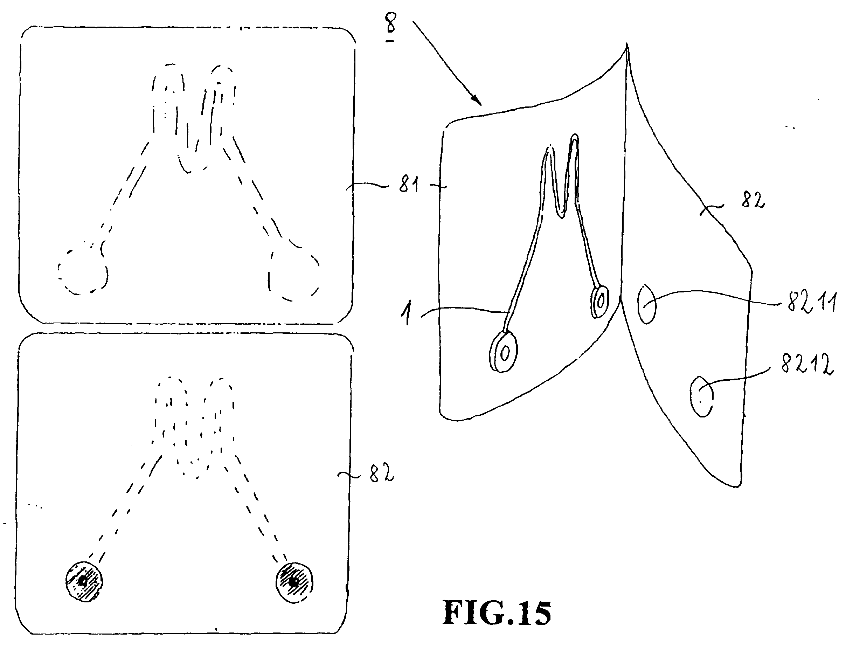

Weiterhin weist die Trägeranordnung ein Befestigungselement 4, 3, 5, 4', 3' auf. Mit diesem Befestigungselement läßt sich die Vorrichtung an einem Kleidungsstück (z.B. Unterwäsche, Damenslip) befestigen. Das Befestigungselement läßt sich z.B. an einem Gürtel, an einem Band oder dergleichen befestigen. Ganz allgemein gilt, daß das Befestigungselement an einem Element befestigbar ist, das an oder in der Nähe eines zu therapierenden Körperteils anbringbar ist. Figur 4 zeigt die Befestigung an einem gestrichelt gezeichneten Textilträger. Wie in den Figuren 14 und 15 dargestellt ist, können die elektropolarisierten Elemente in Taschen 9; 10-1 und 10-2 (Figur 14) bzw. 8 (Figur 15) angeordnet sein.

Bei den in den Figuren 1 bis 13 dargestellten Ausführungsbeispielen ist das Verbindungselement zugleich als das Befestigungselement ausgebildet, wobei das Befestigungselement als Klemme (Klipp) ausgeformt ist. Bei diesen Ausführungsformen ist die Trägeranordnung mit Verbindungs- bzw. Befestigungselement und mit elektropolarisierten Elementen einstückig ausgebildet. Die Gesamtanordnung kann aber auch zwei- bzw. mehrstückig ausgebildet sein.

Das Befestigungselement bzw. die Klemme besteht aus Draht, der unterschiedlich ausgeformt sein kann:

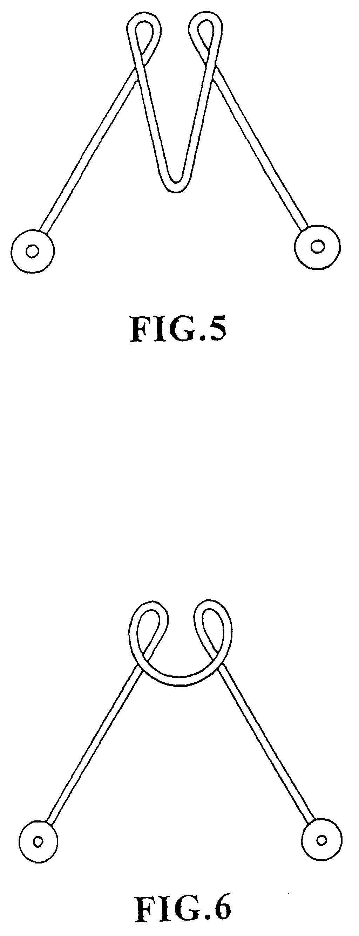

Bei den in den Figuren 1 und 13 dargestellten Ausführungsformen hat das Befestigungselement die Form eines M und bei der in Figur 5 dargestellten Ausführungsform hat das Befestigungselement die Form eines V.

Die in Figur 1 dargestellte Trägeranordnung weist zwei äußere Strangelemente 2, 2' auf, die in einem oberen Bereich eine erste gekrümmte Ausformung 3, 3' aufweisen. An die äußeren Strangelemente 2, 2' schließen sich innere Strangelemente 4, 4' an, die in einem inneren Bereich eine zweite gekrümmte Ausformung 5 aufweisen und dort verbunden sind. An den freien Enden der äußeren Strangelemente 2, 2' sind die zwei elektropolarisierten Elemente bzw. Elektropolarisatoren 6, 6' zum Beispiel mittels Verlöten, Verschrauben, Verschweißen oder Verkleben angeordnet, wobei die Elemente 6, 6' jeweils eine mittige Öffnung 7, 7' haben.

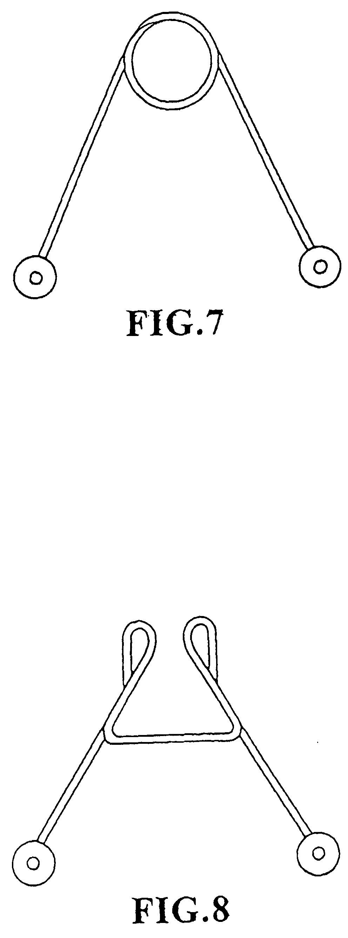

Wie in den Figuren 6, 7 und 11 dargestellt, kann das Befestigungselement ein kreisförmiges Segment aufweisen; bei dem in Figur 7 dargestellten Ausführungsbeispiel hat das Befestigungselement die Form einer Spule.

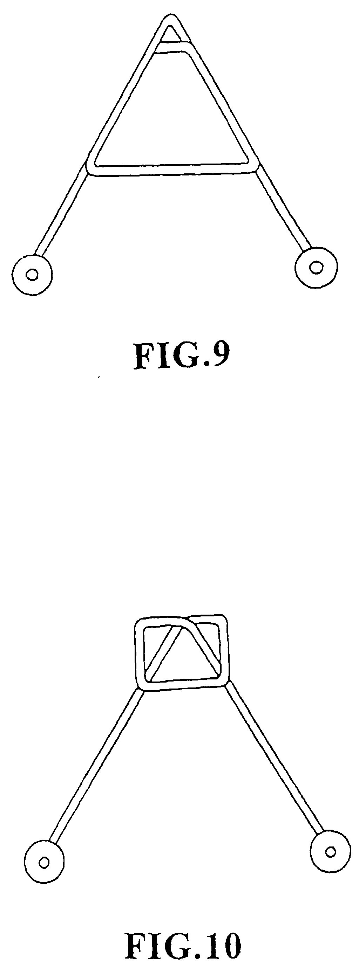

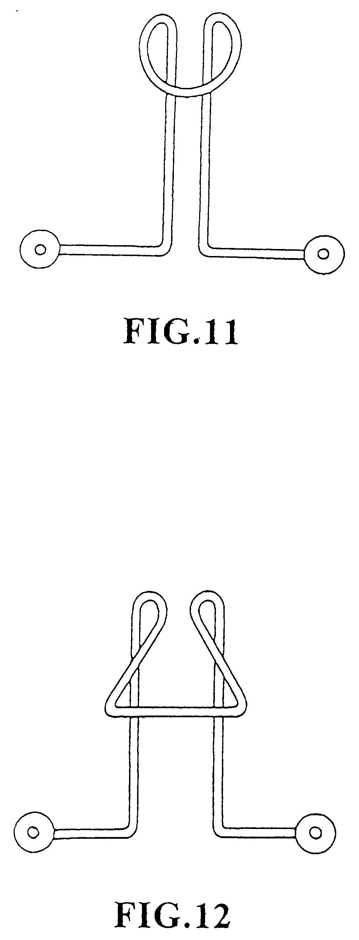

Das Befestigungselement kann auch mindestens ein gerade ausgebildetes Teil aufweisen, wie dies in den Figuren 8, 9, 10, 12 dargestellt ist. So hat das in den Figuren 8 und 12 dargestellte Befestigungselement die Form eines oben offenen Dreiecks, während das in den Figuren 9 und 10 dargestellte Befestigungselement die Form eines Dreiecks bzw. eines Rechtecks hat. Den vorstehend genannten Befestigungselementen, die in den Figuren 8, 9, 10, 12 dargestellt sind, ist gemeinsam, daß sie mindestens zwei gerade ausgebildete, zueinander abgewinkelte Segmente aufweisen.

Wie in Figur 1 dargestellt, sind die elektropolarisierten Körper 6, 6' über je einen Schenkel 2, 2' mit dem Verbindungs- bzw. Befestigungsteil verbunden. Die Schenkel 2, 2' können zum Beispiel schräg gegenüber dem Verbindungs- bzw. Befestigungsteil abgewinkelt sein (siehe Figuren 1, 5 bis 10) oder alternativ hierzu im wesentlich rechtwinklig gegenüber dem Verbindungs- bzw. Befestigungsteil abgewinkelt sein (siehe Figuren 11 bis 13). Die Abstände (d1 in Figur 1) zwischen den elektropolarisierten Elementen sind bei allen Ausführungsformen (Figuren 1 bis 15) im wesentlichen gleich.

Die in den Figuren 1 bis 13 dargestellten Befestigungselemente sind lediglich Beispiele; die Befestigungselemente können auch anders ausgeformt sein; Beispielsweise kann ein Befestigungselesment die Form eines U haben.

Während die in den Figuren 1 bis 13 dargestellten Befestigungselemente aus einem Draht gebildet sind, können auch andere Befestigungselemente vorgesehen sein, die zum Beispiel zangenförmig ausgestaltet sind, wobei zwei Zangenelemente mittels mindestens einer Feder gegeneinander gedrückt werden.

Das Verbindungselement, das zwei elektropolarisierte Elemente 6, 6' miteinander verbindet, kann auch durch ein Kleidungsstück bzw. durch einen Teil eines Kleidungsstücks, zum Beispiel den Saum eines Unterwäschestücks gebildet sein. Das Verbindungselement kann Kunststoff und/oder Textilmaterial aufweisen; insbesondere kann das Vorbindungselement ein selbstklebendes Teil aufweisen, das zum Beispiel an einem Kleidungsstück oder direkt an dem zu therapierenden Körperteil befestigt wird. Bei dem oberen in Figur 14 dargestellten Ausführungsbeispiel sind zwei elektropolarisierte Elemente 6, 6' in eine Tasche 9 integriert, wobei die elektropolarisierten Elemente auf eine Taschenseite im Tascheninneren zum Beispiel aufgenäht, aufgeklebt oder in anderer Weise befestigt sind. Auf einer weiteren Taschenseite sind zwei kreisförmige Öffnungen angeordnet, die die elektropolarisierten Elemente freigeben und einen direkten Körperkontakt ermöglichen. Bei diesem Ausführungsbeispiel bildet die Tasche bzw. das Taschenmaterial zwischen den beiden elektropolarisierten Elementen das Verbindungselement. Die elektropolarisierte Elemente 6, 6' sind mit einem starren oder nicht starren Draht 2'' verbunden.

Die Trägeranordnung besteht aus Metall, insbesondere Stahl, insbesondere Austinitstahl; und/oder aus Kupfer. Die elektropolarisierten Elemente 6, 6' können mit einer Edelmetallschicht, insbesondere mit einer Goldschicht überzogen sein.

Mindestens eines der elektropolarisierten Elemente 6, 6' kann als Zylinderring ausgeformt sein. Die elektropolarisierten Elemente 6, 6' können auch eine andere geometrische Form, beispielsweise die Form einer Kugel haben.

Die in den Figuren dargestellten elektropolarisierten Elemente 6, 6' sind mehr als vier Zentimeter, insbesondere etwa 5,4 (oder 6,3) Zentimeter voneinander beabstandet (Abstand d1 in Figur 1). Der Abstand d2 (Figur 1) der Mittelpunkte der Zylinderringe beträgt etwa 6,4 (oder 7,3) Zentimeter. Der Außendurchmesser dr des Zylinderrings (Figur 1) beträgt etwa 1 Zentimeter, wobei die Breite br des Zylinderrings etwa 3,3 Millimeter beträgt. Der Zylinderring hat eine Höhe von etwa 5 Millimeter. Die elektropolarisierten Elemente 6, 6' können jedoch auch andere Abmessungen und Formen aufweisen, beispielsweise können sie einen viereckigem, ovalen oder andersförmigen Querschnitt aufweisen.

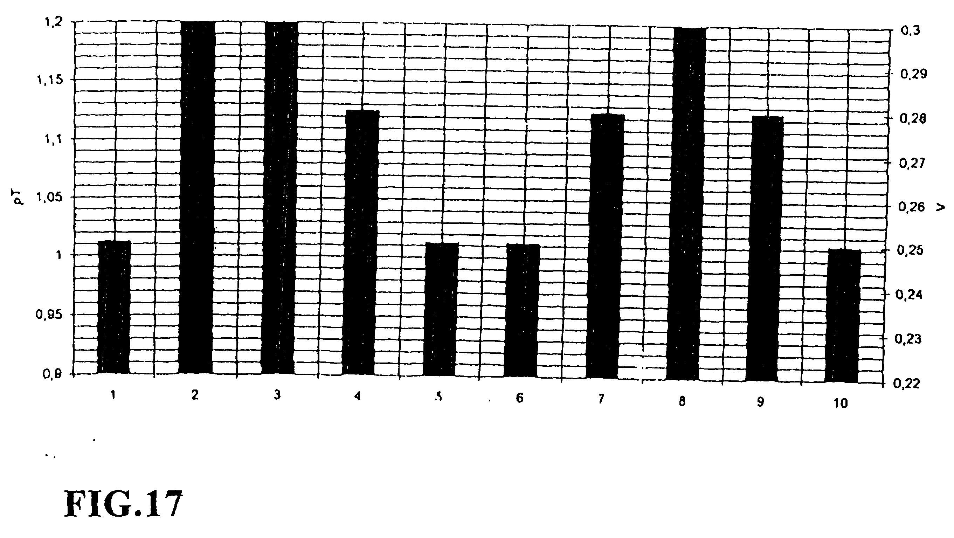

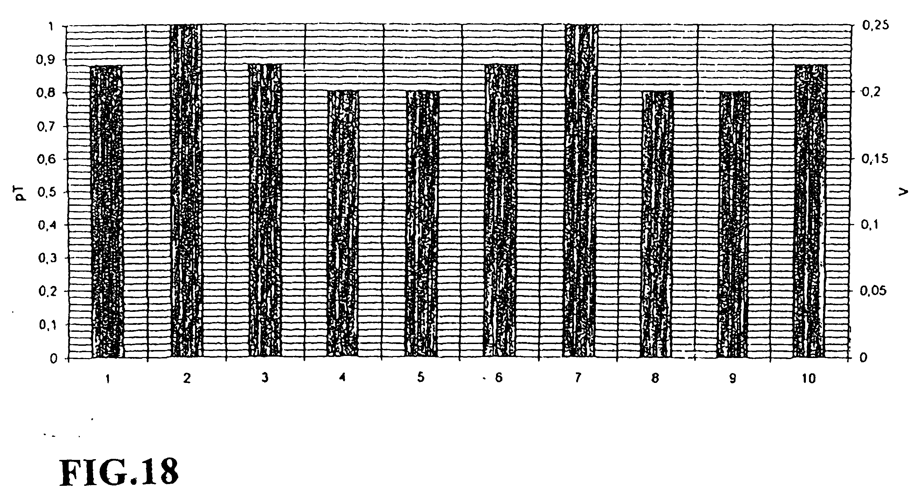

Die magnetische Ausgestaltung der erfindungsgemäßen Trageranordnungen ergibt sich zum Beispiel aus den Figuren 17 und 18: Die Werte der magnetischen Flußdichte liegen in der Größenordnung von 1 Picotesla und damit in der Größenordnung der magnetischen Flußdichte, die von elektrischen Strömen des Herzens, von Muskeln, Nerven, Gewebe und Sehnen gebildet werden.

Die magnetische Flußdichte beträgt insbesondere nicht mehr als 500 Gauß (1 Tesla = 10.000 Gauß).

Die erfindungsgemäßen Trägeranordnungen können aber auch stärke Magneten aufnehmen, die beispielsweise in die Öffnungen 7, 7' (Figur 1) eingesetzt werden. Damit lassen sich andere therapeutische Ziele erreichen (Heilung bei Knochenbrüchen, Reduzierung bestimmter Knochenleiden durch bessere Verfestigung von Kalzium).

Eine weitere Ausgestaltung der Trägeranordnung, die zum Beispiel in Figur 14 unten dargestellt ist, weist ein elektropolarisiertes Element auf, das zum Beispiel mit einem Textilträger 10-1 bzw. 10-2 fixiert ist. Dieser bildet ein Befestigungselement, das eine Befestigung des elektropolarisierten Elements mit einem weiteren elektropolarisierten Element, mit einem weiteren Befestigungselement, mit einem Kleidungsstück und/oder mit einem Element ermöglicht, das an oder in der Nähe eines zu therapierenden Körperteils anbringbar ist.

Die erfindungsgemäße Trägeranordnung kann weiterhin wie in Figur 15 dargestellt in eine Tasche 8 integriert sein. Die Tasche weist ein erstes Taschenteil 81 auf, an dem die Trägeranordnung 1 fixiert ist und das dem zu therapierenden Körperteil abgewandt ist. Weiterhin weist die Tasche ein zweites Taschenteil 82 auf, das dem zu therapierenden Körperteil zugewandt ist und zwei Öffnungen 8211 und 8212 aufweist, die die elektropolarisierten Körper freigeben und Kontakt mit dem zu therapierenden Körperteil ermöglichen.

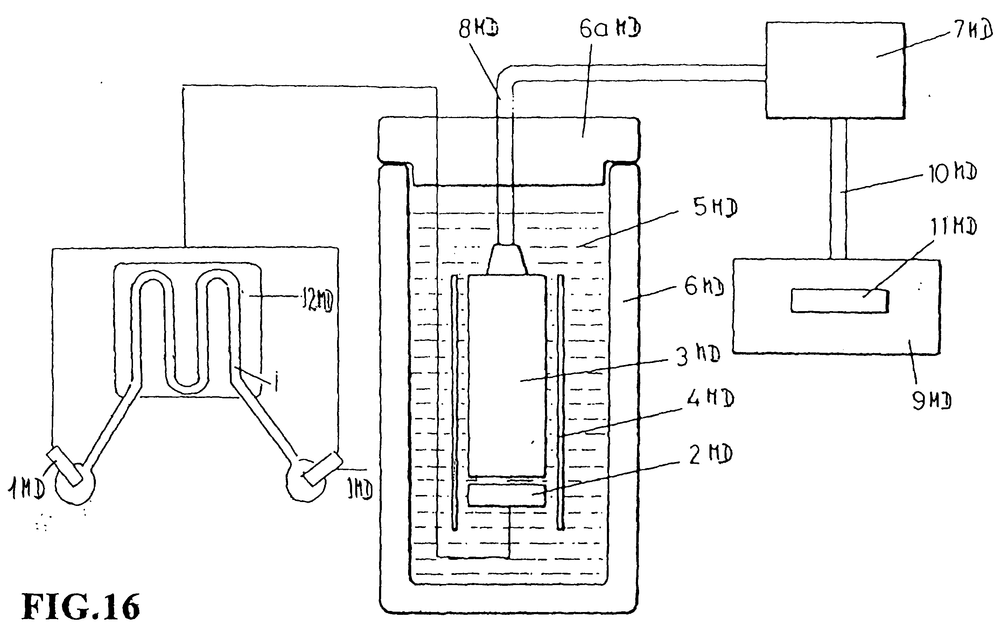

Figur 16 zeigt eine Anordnung, ein Biomagnetometer 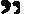 Superconducting Quantum Interference Device (SQUID)", das zum Beispiel in den US-Patenten 5,269,746 (Jacobson) und 5,453,072 (Anninos et. al.) beschrieben ist und -mit dem die erfindungsgemäßen Trägeranordnungen 1 gemäß Figur 1 gemessen wurden.

Dabei bedeutet:
- 1: Prüfling, Trägeranordnung 1
- 1MD: Elektrode
- 2MD: Hilfsspule mit 10 Windungen
- 3MD: 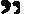 Superconducting Quantum Interference Device (SQUID)"-Detektor
- 4MD: Abschirmung hoher Permeabilität
- 5MD: flüssiger Stickstoff
- 6MD: Dewar-Behälter zur Kühlhaltung des flüssigen Stickstoffs
- 6aMD: thermisch isolierende Abdeckung von 6MD
- 7MD: Kopplungseinheit, Typ 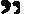 flux locked loop"
- 8MD: Kryoskopkabel
- 9MD: Anzeigeeinheit
- 10MD: Glasfaserkabel
- 11MD: Bildschirmeinheit zur Dantellung gemessener Felder
- 12MD: Wechselmagnetfeld-Generator

Die Ergebnisse dieser Messungen an zwei erfindungsgemäßen Trägeranordnungen nach Figur 1 sind in den Figuren 17 und 18 dargestellt. Es wurden jeweils 10 Messungen an ersten Trägerordnungen (Figur 17) und an zweiten Trägeranordnungen (Figur 18) durchgeführt (horizontale Achse: 1 ... 10 Messungen). Die Werte der magnetischen Flußdichte (Induktion) sind in Picotesla (pT) (linke vertikale Asche angegeben, während die entsprechende Spannung in Volt (V) (rechte vertikale Achse) angegeben ist.

Die ersten und die zweiten Trägeranordnungen unterscheiden sich durch ihre Abmessungen, insbesondere durch den Abstand (d1 in Figur 1) ihrer elektropolarisierten Elemente 6, 6'. Die gemessenen Werte geben Herstellungstoleranzen der verwendeten ersten und zweiten Trägeranordnungen wieder.

Die Werte der magnetischen Flußdichte liegen in der Größenordnung von 1 Picotesla.

Das Instituto de Bioelectromagnetismo Alonso de Santa Cruz, Madrid/Spanien, hat die in Figur 1 dargestellte Trägeranordnung an Frauen getestet. Dabei ergab sich, daß bei 90 Prozent der getesteten Frauen Menstruationsachmerzen verschwanden.

Die Erfindung betrifft auch ein elektropolarisiertes Element', ein Verbindungselement, ein Befestigungselement und/oder eine Tasche für die vorstehend beschriebenen Trägeranordnungen.

| **Bezugszeichenliste** | |
|---|---|
| 1 | Trägeranordnung |
| 2, 2' | äußeres Strangelement, Schenkel |
| 2'' | starres oder nichtstarres, metallisches Verbindungselement (Figur 14) |
| 3, 3' | erster gekrümmter Bereich |
| 4, 4' | inneres Strangelement |
| 5 | zweiter gekrümmter Bereich |
| 6, 6' | elektropolarisierter Körper, Elektropolarisator |
| 7, 7' | mittige Öffnung |
| 8 | Tasche |
| 81 | erstes Taschenteil (auf der dem zu therapierenden Körper abgewandten Seite) |
| 82 | zweites Taschenteil (auf der dem zu therapierenden Körper zugewandten Seite) |
| 8211, 8212 | Öffnungen in 82 |
| 9 | Tasche, Gürtel, Band |
| 10-1, 10-2 | Tasche, Gürtelteil, Bandteil |

| (zu Figur 16) | |
|---|---|
| 1 | Prüfling, Trägeranordnung 1 (siehe oben) |
| 1MD | Elektrode |
| 2MD | Hilfsspule mit 10 Windungen |
| 3MD | 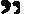 Superconducting Quantum Interference Device (SQUID)"-Detektor |
| 4MD | Abschirmung hoher Permeabilität |
| 5MD | flüssiger Stickstoff |
| 6MD | Dewar-Behälter zur Kühlhaltung des flüssigen Stickstoffs |
| 6aMD | thermisch isolierende Abdeckung von 6MD |
| 7MD | Kopplungseinheit, Typ 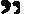 flux locked loop" |
| 8MD | Kryoskopkabel |
| 9MD | Anzeigeeinheit |
| 10MD | Glasfaserkabel |
| 11MD | Bildschirmeinheit zur Darstellung gemessener Felder |
| 12MD | Wechselmagnetfeld-Generator |

## Patentansprüche

1. Trägeranordnung (1) für elektropolarisierte Elemente, insbesondere für therapeutische Zwecke zur äußeren Anwendung
**dadurch gekennzeichnet,**
daß die Trägeranordnung zwei elektropolarisierte Elemente (6, 6') aufweist und daß die Trägeranordnung mindestens ein Verbindungselement (2, 4, 3, 5, 4', 3', 2') aufweist, das die elektropolarisierten Elemente (6, 6') miteinander verbindet.

2. Trägeranordnung nach Anspruch 1, dadurch gekennzeichnet, daß die Trägeranordnung ein Befestigungselement (4, 3, 5, 4', 3') aufweist, mit dem die Vorrichtung an einem Kleidungsstück und/oder an einem Element befestigbar ist, das an oder in der Nähe eines zu therapierenden Körperteils anbringbar ist.

3. Trägeranordnung nach Anspruch 2, dadurch gekennzeichnet, daß das Verbindungselement als das Befestigungselement ausgebildet ist.

4. Trägeranordnung nach Anspruch 2 oder 3, dadurch gekennzeichnet, daß das Befestigungselement als Klemme ausgeformt ist.

5. Trägeranordnung nach einem der vorstehenden Ansprüche, dadurch gekennzeichnet, daß sie einstückig ausgebildet ist.

6. Trägeranordnung nach einem der Ansprüche 4 oder 5, dadurch gekennzeichnet, daß das Befestigungselement aus Draht besteht.

7. Trägeranordnung nach einem der Ansprüche 2 bis 6, dadurch gekennzeichnet, daß das Befestigungselement die Form eines M (Figuren 1, 13) hat.

8. Trägeranordnung nach einem der Ansprüche 2 bis 7, dadurch gekennzeichnet, daß das Befestigungselement die Form eines U oder eines V (Figur 5) hat.

9. Trägeranordnung nach einem der Ansprüche 2 bis 8, dadurch gekennzeichnet, daß das Befestigungselement ein kreisförmiges Segment (Figuren 6, 7, 11) und/oder mindestens ein gerade ausgebildetes Teil (Figuren 8, 9, 10, 12) aufweist.

10. Trägeranordnung nach Anspruch 9, dadurch gekennzeichnet, daß das Befestigungselement mindestens zwei gerade ausgebildete, zueinander abgewinkelte Segmente (Figuren 8, 9, 10, 12) aufweist.

11. Trägeranordnung nach einem der Ansprüche 6 bis 10, dadurch gekennzeichnet, daß der Draht starr oder dehnbar ist.

12. Trägeranordnung nach Anspruch 1, dadurch gekennzeichnet, daß das Verbindungselement Teil eines Kleidungsstücks ist.

13. Trägeranordnung nach Anspruch 1 oder 12, dadurch gekennzeichnet, daß das Verbindungselement Kunststoff und/oder Textilmaterial aufweist.

14. Trägeranordnung nach Anspruch 1, dadurch gekennzeichnet, daß das Verbindungselement ein selbstklebendes Teil aufweist.

15. Trägeranordnung nach einem der Ansprüche 1 bis 11, dadurch gekennzeichnet, daß sie mindestens teilweise aus Metall, insbesondere Stahl, insbesondere Austinitstahl und/oder aus Kupfer besteht.

16. Trägeranordnung nach einem der vorstehenden Ansprüche, dadurch gekennzeichnet, daß mindestens eines der elektropolarisierten Elemente (6, 6') als Zylinderring ausgeformt ist.

17. Trägeranordnung nach einem der vorstehenden Ansprüche, dadurch gekennzeichnet, daß die elektropolarisierten Elemente (6, 6') mehr als vier Zentimeter, insbesondere etwa 5,4 Zentimeter voneinander beabstandet sind.

18. Trägeranordnung nach einem der vorstehenden Ansprüche, dadurch gekennzeichnet, daß die magnetische Flußdichte der elektropolarisierten Elemente (6, 6') nicht mehr als 500 Gauß beträgt.

19. Trägeranordnung für elektropolarisierte Elemente, insbesondere für therapeutische Zwecke zur äußeren Anwendung
**dadurch gekennzeichnet,**
daß die Trägeranordnung ein elektropolarisiertes Element (6) aufweist und daß die Trägeranordnung mindestens ein Befestigungselement zur Befestigung des elektropolarisierten Elements (6) mit einem weiteren elektropolarisierten Element, mit einem weiteren Befestigungselement, mit einem Kleidungsstück und/oder mit einem Element (10-1, 10-2) aufweist, das an oder in der Nähe eines zu therapierenden Körperteils anbringbar ist.

20. Elektropolarisiertes Element (6, 6') für eine Trägeranordnung (1) nach einem der vorstehenden Ansprüche.

21. Verbindungselement für eine Trägeranordnung (1) nach einem der Ansprüche 1 bis 19.

22. Tasche (8, 9, 10-1, 10-2) zur Aufnahme mindestens eines elektropolarisierten Elements (6, 6') und/oder einer Trägeranordnung (1) nach einem der Ansprüche 1 bis 19.

23. Tasche nach Anspruch 22, dadurch gekennzeichnet, daß die Tasche mindestens eine Öffnung (8211, 8212) im Bereich des mindestens einen elektropolarisierten Elements (6, 6') aufweist.
